# EUROPÄISCHE PATENTSCHRIFT

(11) **EP 0 923 731 B1**
(45) Veröffentlichungstag und Bekanntmachung des Hinweises auf die Patenterteilung: **19.01.2005**
(21) Anmeldenummer: 97942833.1
(22) Anmeldetag: 28.07.1997
(51) Int. Cl.: G01N 33/487, G01N 33/49, G01N 27/28

(54) **VORRICHTUNG ZUR AUSTAUSCHBAREN AUFNAHME VON MESSKARTUSCHEN, BZW. MESSZELLEN, ZUR BESTIMMUNG BIOCHEMISCHER MESSPARAMETER, IN COMPUTERGESTEUERTEN ANALYSENSYSTEMEN**
HOUSING FOR INTERCHANGEABLE MEASUREMENT CARTRIDGES OR CELLS USED FOR SETTING BIOCHEMICAL MEASUREMENT PARAMETERS IN COMPUTER-CONTROLLED ANALYSIS SYSTEMS
DISPOSITIF POUR LOGER DE FACON INTERCHANGEABLE DES CARTOUCHES OU DES CELLULES DE MESURE, POUR DETERMINER DES PARAMETRES DE MESURE BIOCHIMIQUES DANS DES SYSTEMES D'ANALYSE COMMANDES PAR ORDINATEUR

(30) Priorität: 31.07.1996 DE 19630981
(43) Veröffentlichungstag der Anmeldung: 23.06.1999
(73) Patentinhaber: Petermann, Heike, 27283 Verden (DE); Fenzlein, Paul-Gerhard, 90431 Nürnberg (DE)
(72) Erfinder: FENZLEIN, Paul-Gerhard, D-90431 Nürnberg (DE)
(86) Internationale Anmeldenummer: PCT/EP1997/004078
(87) Internationale Veröffentlichungsnummer: WO 1998/005958

(56) Entgegenhaltungen:
- WO-A-85/02257
- WO-A-93/00582

## Beschreibung

Die Erfindung beschreibt eine neuartige Vorrichtung mit Meßkartuschen bzw. Meßzellen zur austauschbaren Aufnahme von Meßkartuschen (5), bzw. Meßzellen (6) in Laboranalysensystem , (1) mit speziell dafür ausgestatteten Aufnahmen (20), die zum einen eine vollautomatische, computergesteuerte Benutzung, sowie die Austauschbarkeit der Meßkartuschen (5), bzw. Meßzellen (6), während der jeweils festgelegten Betriebszeit und der für die einzelnen in den Meßkartuschen (5), bzw. Meßzellen (6), definierten Anzahl von Messungen, erlaubt. Die speziell daraufhin konzipierten Meßkartuschen (5) oder Meßzellen (6), können aufgrund der erfindungsgemäßen Konstruktion während der obengenannten erlaubten Betriebsdauer, außerhalb des Analysengerätes (1) problemlos gelagert werden und darüberhinaus während der entsprechenden Betriebsdauer und den, in den jeweiligen Meßkartuschen (5) oder Meßzellen (6) erlaubten Anzahl von Messungen, in jedem beliebigen, vorgenannten Analysengerät (1), mit der entsprechenden Aufnahme (20) für diese Meßkartuschen (5) und Meßzellen (6) eingesetzt werden.

Nach dem Stand der Technik werden in der medizinischen, biochemischen und pharmazeutischen Labordiagnostik bei der Messung von Elektrolyten, Blutgasen und anderer wichtiger Stoffwechselprodukte, wie Glukose, Laktat, Harnstoff, Kreatinin oder der Leberfunktionswerte, Analysengeräte verwendet, die potentiometrische, bzw. amperometrische Meßelektroden besitzen. Bei bestimmten Meßparametern können auch weiter Meßverfahren, wie z.B. die Messung der Leitfähigkeit, zur Bestimmung der oben genannten Meßparameter verwendet werden.

Die in diesen Geräten bevorzugt verwendete Ausführungsform der Meßzellen ist die sogenannte "Schindler' sche Durchflußmeßzelle", wie sie seit über 20 Jahren von den meisten Anbietern von Elektrolyt- und Blutgas-Analysengeräten verwendet wird.

Diese "Schindler'sche Durchflußmeßzelle" ist im Normalfall modular aufgebaut, so daß sie über die unterschiedliche Bestückung, mit normalerweise 2 bis 8 verschiedenen Meßparametern, Analysengeräte für unterschiedliche Anwendungsbereiche aufgebaut werden können.

Das (Betriebs-) Konzept dieser "Standard-" Analysengeräte, wie sie in der deutschen Patentanmeldung der Firma AVL P 39 41 169.9 "Analysengerät zur Messung flüssiger und gasförmiger Proben" beschrieben ist, beinhaltet jedoch durch den komplexen und damit auch relativ teueren Aufbau der Meßzellen, daß die einzelnen Meßelektroden eine lange Lebensdauer, von mindestens mehreren Monaten bis hin zu einem Jahr, erreichen müssen. Dies bedeutet weiter für das Gerätekonzept, daß die Elektroden während ihrer Einsatzdauer und vor allem während der Meßpausen speziell konditioniert und kalibriert, sowie in regelmäßigen Abständen gewartet werden müssen. Die Kalibirierung und Konditionierung erfolgt über spezielle Flüssigkeiten, bzw. Gase, die als Beutel oder Flaschen im oder am Gerät angebracht sein müssen. Dies bedeutet auch, daß diese Geräte, als Tischgeräte aufgebaut, sehr groß und damit nur schwerlich portabel sind. Ein weiterer Nachteil dieser Gerätekonzeption ist, daß für unterschiedliche Meßaufgaben, entsprechend mehrere dieser teueren Analysengeräte angeschaft werden müssen, oder über einen üblicherweise externen Geräteservice das Gerät, die Meßzellen und das zugehöriges Zubehör, auf die neue Meßaufgabe umgerüstet werden muß.

Alternativ zur vorgenannten Technologie der "Schindler'schen Durchflußmeßzelle" wird in der Labordiagnostik vermehrt auch die sogenannte "Trockenchemie" in Form von Teststreifen bzw.Plättchen oder Einmalkartuschen eingesetzt. Diese Form der Analysengeräte, z.B. das Reflotron® der Firma Boehringer-Mannheim, oder das Kodak-Ektachem® -Verfahren ermöglicht es mit einem Basis-Gerät beim Reflotron®, bzw. mit einem Basis-Gerät und zwei Satellitensystemen beim Kodak-System nahezu alle Meßaufgaben zu bewältigen. Die Problematik, d.h. die Nachteile hierbei liegen in den verwendeten Meßverfahren, der Helmholtz-Kugel beim Reflotron®, oder der Filmherstellung für die Kodak-Analysenplättchen. Die Einzelanalysen sind dabei relativ teuer, zeitlich aufwendig, da die einzelnen Untersuchungen nacheinander abgearbeitet werden müssen (pro Analyse werden zwischen 2 bis 5 Minuten benötigt) und trotzdem ungenauer als die mit den Standardsystemen sind.

Ähnliches trifft auf die in der internationalen Anmeldung WO 85/ 02257 "Clinical Chemistry Analyser", sowie der EPA 0 306 158 "Cartridge with sensor" beschriebene Ausführungsform einer Einmal-Meßzelle und dem zugehörigen Analysensystem zu. Auch hier können unterschiedliche Meßkartuschen mit unterschiedlichen Parameterprofilen (ca. 1-4 unterschiedliche Parameter pro Kartusche) mit einem einzigen Analysensystem gemessen werden. Der Nachteil dieser Ausführungsform ist zum einen, daß die Kartuschen jeweils nur einmal genutzt werden können, was ökonomisch durch den relativ hohen Preis der Meßkartusche, sowie ökologisch, durch die große Zahl von zu entsorgenden Kartuschen, von Nachteil ist, und, zum anderen daß das Meßverfahren über die Differenzmessung ungenauer ist als die direkte Messung gegen eine Bezugs-, bzw. Referenzelektrode.

Allen vorgenannten Systemen ist gemeinsam, daß die austauschbaren Meßzellen, Meßstreifen, Meßplättchen und -Kartuschen über Codierungen mittels Barcodes spezielle Informationen über die verwendeten Parameter der Meßzellen, Meßstreifen, Meßplättchen und -Kartuschen dem Gerät mitteilen und somit die Bedienung der jeweiligen Geräte vereinfachen und die Benutzung sicherer gestalten.

Im Bereich der Labordiagnostik wird in der europäischen Patentanmeldung 0 544 237A "Vorrichtung zum Messen von Ionenkonzentrationen in fließfähigen Medien" ein Analysensystem mit austauschbaren Meßeinheiten beschrieben, wobei die Meßeinheiten für unterschiedliche Anwendungsgebiete mit unterschiedlichen Meßzellen, die ihrerseits mehrere, miniaturisierte Meßelektroden und eine separate ebenfalls minaturisierte integrierte Bezugselektrode beinhaltet. Dieses Konzept ist daher so vorteilhaft, da es die Vorteile der "Schindlers schen Durchflußmeßzelle", mit der hohen Meßgenauigkeit, nutzt, gleichzeitig durch das Konzept der beschränkten Nutzungsdauer, kein Einmal-Artikel, und über die Kartuschentechnik, das System vielseitig einsetzbar macht. Dadurch, daß alle wesentlichen Sensor-, Elektroden- und Kartuscheninformationen auf einem elektronischen Speichermedium stehen, können diese Meßkartuschen mehr Informationen für eine einfachere Benutzung tragen, was es erlaubt, diese Systeme für mehrere Messungen über einen bestimmten Zeitraum, auch in anderen Geräten, während der festgelegten Nutzungsdauer, einzusetzen. Dieses Gerätekonzept beinhaltet aber, daß zumindest eine halbautomatische Kalibrierung und Spülung der Meßzelle zwischen den einzelnen Messungen durch den Benutzer erfolgt. Dies muß über die im Ausführungsbeispiel gezeigten Probenaufnahme sowohl für die eigentliche Meßprobe, wie auch für die entsprechenden Spül- und Kalibrierungslösungen erfolgen.

Die vorliegende Erfindung beschreibt eine Vorrichtung für die Schnittstelle zwischen dem Analysengerät (1) und einer austauschbaren - und in diesem Falle "intelligenten Meßkartusche (5) bzw. Meßzelle (6)" -, sodaß über die in der EPA 41 39 121 beschriebenen Ausführung hinaus der große Vorteil besteht, daß die entsprechenden Strömkanäle (7, 9, 10, 12) in der Meßzelle (6) bei der Lagerung außerhalb des Analysengerätes (1) durch spezielle (Gummi-) Septen (13) völlig hermetisch dicht abgeschlossen sind und erst wenn die Meßkartusche (5) bzw. Meßzelle (6) in das Gerät (1) bzw. in die definierte Ventilschnittstellen (2, 14) eingeführt wird, über die in der Ventilschnittstellen (2, 14) integrierten und gegenüber dem Benutzer geschützen Kanülen (3) angestochen wird.

Dieses Prinzip, daß Kanülen (3) diese speziellen Septen (13) anstechen, ermöglicht eine reversible Verbindung zwischen Ventilblock (2, 14), Pumpe (15) und der austauschbaren Meßkartusche (5) bzw. Meßzelle (6). Die Strömkanäle (7, 9, 10, 12) können hierbei aus gespritzen Kunststoff-, z.B. PVC-Teilen aufgebaut werden. PVC ist für Meßzellen (6) mit einer beschränkten Betriebsdauer, z B. 100 Messungen innerhalb 7 bis 14 Tagen - extrem kostengünstig und erlaubt, daß die aktive Meßzelle (10) mit dem zugehörigen Strömkanal (10) hoch minaturisiert werden kann. Gleichzeitig ermöglicht dies eine viel präzisere Positionierung der Proben- und Spül- bzw. Kalibrierlösungsvolumina im aktiven Strömkanal (10) der Meßzelle (6), als die in den bisherigen Analysensystem benutzten PVC-Verbindungsschläuchen.

Weiter wird dadurch das gas- und wasserdampfdichte System in der Meßkartusche (5), bzw. der Meßzelle (6) geschaffen, in dem über den Lagerungszeitraum und während des Betriebszeitraumes leicht unterschiedliche Medien (Flüssigkeiten und, oder Gase) in den Meßkanal (10) eingebracht werden können.

Dies ist ein ganz wesentlicher Vorteil gegenüber einem bereits auf dem Markt befindlichem Analysensystem GemPremier® der Firma Mallinckrodt, das nur eine einzige Meßkartusche mit 6 über einen Strichcode definierten Meßparametern, für Kalium, Natrium, Calcium, pH, sowie den Blutgasen pO₂ und pCO₂ hat, aber nach der ersten Messung, d.h. während der Betriebsdauer von mehr als 50 Messungen / Kartusche, nicht mehr aus dem Gerät herausgenommen werden kann, da die Meßzelle nicht mehr dicht ist. Innerhalb der Meßkartusche sind neben der eigentlichen Meßzelle, die über eine Leiterkarte elektrisch mit dem Analysengerät verbunden ist, die für die Messungen benötigten Kalibrier- und Spüllösungen, sowie der Abfallbeutel mit üblichen PVC-Schläuchen verbunden. Dieses komplexe Innenleben der Kartusche wird über eine normale Rollerpumpe, die ebenfalls in der Kartusche integriert ist, lediglich über eine meachnische Welle von außen betrieben, und ist daher ein Grund für Probleme mit der Dichtigkeit der gesamten Kartusche und somit des allgemeinen Betriebes.

in der hier beschriebenen Erfindung werden diese Nachteile des vorgenannten Analysengerätes durch die bereits beschriebene Schnittstelle des Ventil- (2, 14) und Pumpenblocks (17) auf der Geräteseite und der Meßkartusche (5) bzw. und, oder der Meßzelle (6), mit den, durch spezielle Septen (13) abgeschlossenen Strömkanälen (7, 9, 10, 12), beseitigt. Diese werden von der Geräteseite her durch die Kanülen (3) angestochen und kontaktiert.

Durch die Verwendung von besonders präzisen, hydraulischen Pumpen (17), mit einer Pumpgenauigkeit von ±1µl, können mit diesem Konzept eines hochpräzisen, miniaturisierbaren Strömkanats (7, 9, 10, 12), bis minimal 70 µl, einem intelligenten Speicher (16) in der Meßkartusche (5), nicht nur unterschiedliche Meßkartuschen (5) mit unterschiedlicher Parameterbestückung, sondern für den Einsatz vor allem im Bereich der Frühgeborenenmedizin, durch die Kombination aller obengenannten Faktoren, unterschiedliche Probenvolumina bis ≤ 100 µl, problemlos und exakt im Strömkanalanteil der aktiven Meßzelle (10) positioniert werden. Die entsprechenden Daten für die jeweilige Pumpensteuerung werden vom Speicher (16) der Kartusche (5) lediglich ausgelesen und umgesetzt.

Durch das Prinzip der getrennten, über die Septen (13) abgeschlossenen Strömkanäle (7, 9, 10, 12) ist die hermetische Abgrenzung- der einzelnen Spül- und Kalibrierungslösungen untereinander, sowie vom eigentlich aktiven Teil, im Bereich (10) der Meß- und Referenzelektroden (11), und vom Abfallbehältnis gegeben.

Die Proben selbst werden direkt über eine spezielle Probenaufnahme (4) im Ventilblock (2) eingebracht Diese Probenaufnahme (4) ist beim Einbringen der Meßkartusche (5) oder der Meßzelle (6), auf den Ventil- (2, 15) und Pumpenblock (17) im Analysengerät (1), durch eine Öffnung (8) in der Meßkartusche (5) oder der Meßzelle (6), von außen vom Benutzer leicht mit einer Spritze (24) oder einer Kapillarkanüle (24) erreichbar. Ober die entsprechende Meß- und Betriebssoftware wird die Probe dann vollautomatisch über die vorgenannte Pumpensteuerung in den aktiven Teil des Strömkanals (10), in die Meßzelle (6), gezogen.

Diese Art der erfindungsgemäßen Vorrichtung erlaubt den Einsatz solcher Meßsysteme (1) in vielen weiteren Bereichen, z B. in voll-µ-Prozessorgesteuerten Meß-Apparaturen, für die Steuerung von Dialyse oder von Herz-Lungen-Maschinen, durch die Integration von sterilen Sensoren (11), wie sie in der EPA 0 302 228 "Sensor zur Messung der Aktivität von lonen, Verfahren zu dessen Herstellung, sowie Sensoren und zugehörige Anordnung" in die erfindungsgemäßen Meßkartuschen (5) und Meßzellen (6, 37). Hierbei werden die sterilen Meßzellen (6, 37) über eine weitere spezielle Ausführungsform der erfindungsgemäßen Meßelektrodeneinsatz (38) über eine Sterilschleuße (34) in die Kalibrierungskammer (35) eingebracht. Ober eine weitere Sterilschleuße (34) in dieser Meßzelle (5, 37) kann der sterile Meßelektrodeneinsatz (38) z. B. in das Extra-Korporale-(Schlauch-)System (36) einer Dialyse-, Filtrations-, bzw. Herz-Lungen- eingebracht werden. Diese Vorrichtung erlaubt darüberhinaus den Einsatz solcher Meßzellen (5, 37) oder auch in den Bereichen der Biotechnologie, sowie der pharamzeutischen Technik.

Weitere Einzelheiten und Vorteile der Erfindung sind der nachfolgenden Beschreibung entnehmbar, in der ein Ausführungsbeispiel anhand der Figuren näher erläutert wird.

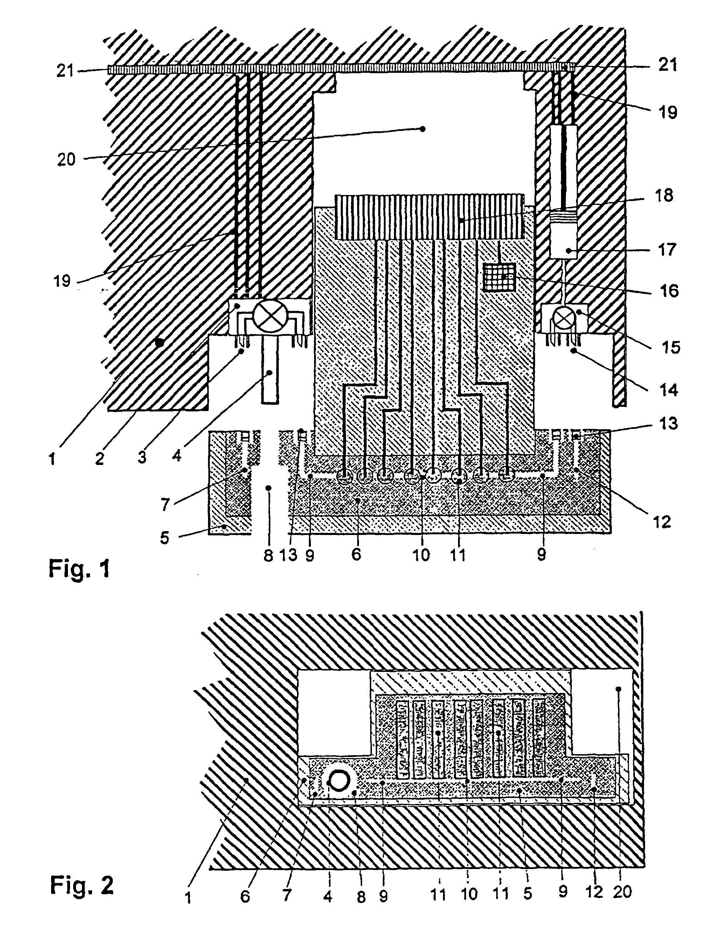

Es zeigen
- **Fig.1**: eine Aufsicht auf die Ebene der Schnittsstelle der Meßkartuschenaufnahme (20), mit Ventil- und Pumpblöcken (2, 15) in einem teilweise, schematisch dargestellten Analysengerät (1) mit integrierter Rechnerelektronik, - und der Meßkartusche (5), mit über Septen (13) abgeschlossenen Strömkanälen (7, 9, 12).
- **Fig.2**: schematisch einen Teil der Frontansicht eines Analysengerätes (1) mit einer in der Meßkartuschenaufnahme (20) befindlichen Meßkartusche (5).

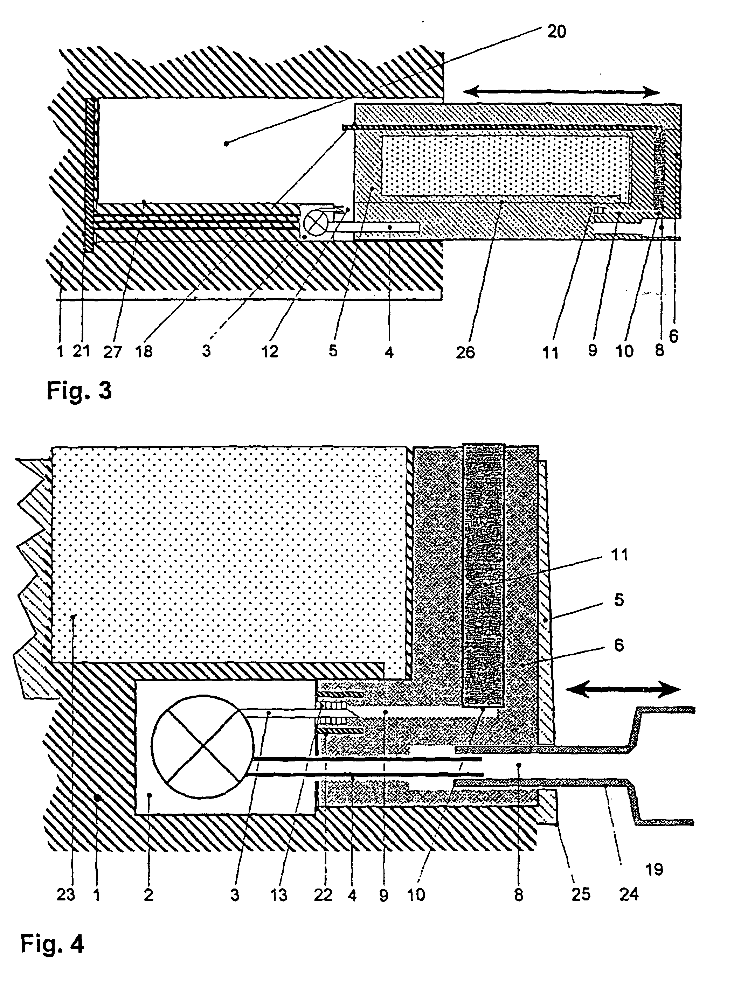

- **Fig. 3**: die Anordnung der jeweiligen Schnittstelle des Ventilblocks (2) mit der integrierten Probenaufnahme (4), bezogen auf die an der seitlichen Frontseite der Meßkartusche (5) befindlichen Septen (13) als Kontaktstelle des Strömkanals (9) der aktiven Meßzelle (10), in der Meßzelle (6), sowie die Durchtrittsöffnung (8) für die im Ventilblock (2) integrierte Probenaufnahme (4) durch die Meßkartusche (5() und deren Meßzelle (6).
- **Fig. 4**: eine Detaildarstellung der Ventilblock- / Meßzellenschnittstelle.

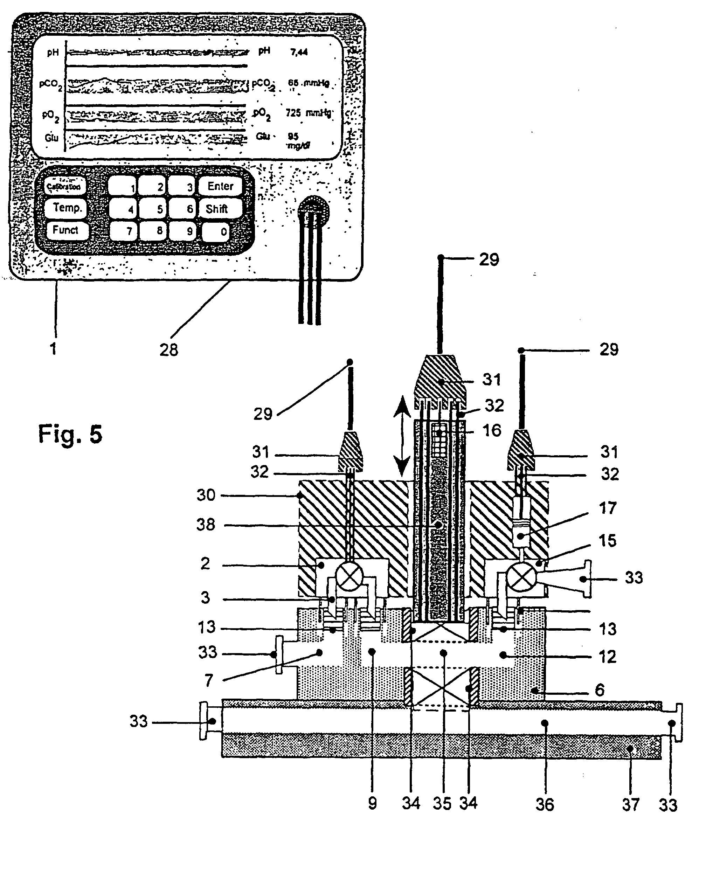

- **Fig. 5**: eine Variation der Vorrichtungen aus den Fig. 1 bis 4, die die Aufnahme (30) für eine modifizierte Meßzelle (6, 37) außerhalb des Analysengerätes (1) hat und deren Aufnahme (30) mit den Ventilblöcken (2, 15) und dem separaten Meß- und Bezugselektrodeneinsatz (38) über Kabel (29) mit dem Analysengerät (1) verbunden ist.

Die Fig. 1 zeigt einen Querschnitt durch eine erfindungsgemäße Ausführungsform der speziellen Aufnahmevorrichtung (20), die zur austauschbaren Aufnahme von Meßkartuschen (5) dient. In diesen Meßkartuschen (5) befinden sich in der Meßzelle (6) Strömkanäle (7, 9, 10, 12) mit Septen (13), durch die diese Strömkanäle (7, 9, 10) hermetisch dicht abgeschlossen werden. Beim Einführen dieser Meßkartuschen (5) in computergesteuerte Analysensysteme (1), die in den Fig. 1 bis 4 lediglich über die Schraffuren schematisch angedeutet sind, werden die Septen (13) in den Strömkanälen (7, 9, 12) über Kanülen (3, 14) angestochen, die in den Frontseiten der Ventilblöcke (2, 15) geschützt integriert sind.

Damit werden die in der Meßkartusche (5) und, bzw. oder in der Meßzelle (6) über die verschiedenen Strömkanäle (7, 9, 12) eingebundenen Teile, wie die Behälter (23) für Spüllösungen, Kalibrierlösungen und der Abfallbehälter, mit dem eigentlichen aktiven Teil des Strömkanals (10), in dem sich die Meß- und Bezugselektroden (11) befinden, verbunden. Ober die beiden Ventilblöcke (2, 15) - in besonderen Ausführungsformen können dies auch mehr als 2 Ventilblöcke sein - werden Ober die mit dem Ventilblock (15) angebundene Pumpe (17), die Spül- bzw. Kalibrierlösungen über den Strömkanal (7) aus einem der entsprechenden Behältnisse (23) der Meßkartusche (5) in den aktiven Teil des Strömkanals (10) in die Meßzelle (6) mit den Meß- und Bezugselektroden (11) eingezogen und positioniert. Über die im Ventilblock (2) integrierte Probenaufnahme (4), die, wenn die Meßkartusche (5) in die (Meßkartuschen-) Aufnahme (20) eingeschoben ist, durch die in der Meßkartusche (5) und der Meßzelle (6) vorgesehenen Öffnung (8) von außen erreichbar ist, wie detaillierter in der Fig. 4 dargestellt, wird aus Spritzen bzw. Kapillaren (24) die entsprechende Probe ebenfalls über die Pumpe (17) über den Strömkanal (9) in den aktiven Strömkanal (10) unter die Meß- und Bezugselektroden (11) eingezogen und richtig positioniert.

Damit der Meßablauf für den Benutzer möglichst vollautomatisch und vom Analysengerät (1) mit integrierter Rechnerelektronik gesteuert ablaufen kann, müssen die wichtigen Informationen über die verwendete Meßkartusche (u. a. Art und Anzahl der Meßparameter, benötigtes Probenvolumen, elekrochemische Kalibrierungs- und Herstellungsdaten der einzelnen Meßelektroden, Herstellungsdatum, Lebens- und Betriebsdauer der Meßkartusche (5) bzw. der Meßzelle (6), Anzahl der (noch) erlaubten Anzahl der Messungen, usw.) individuell in jeder der Meßkartuschen (5) oder Meßzellen (6) abgespeichert sein.

In der Fig. 1 wird, gezeigt wie über ein elektronisches, les- und beschreibbares Speichermedium (16) bei eingeschobener Meßkartusche (5) bzw. Meßzelle (6) über den Steckerkontakt (18) die obengenannten Daten über das Gegenstück (21) des Steckkontaktes (18) der im Analysengerät (1) integrierten Rechnerelektronik zur Verfügung gestellt werden und entsprechende Daten der Auswerteelektronik auf das Speichermedium (16) rückgeschrieben werden können.

Die Ventilblöcke (2, 15) sowie die Pumpe (17) in der Meßkartuschenaufnahme (20) des Analysengerätes (1) werden über entsprechende Steuerleitungen (19) elektrisch von der Rechnerelektronik des Analysengerätes (1) gesteuert.

Die Fig. 2 zeigt die schematische Teil-Frontansicht eines Analysensystems (1) mit der erfindungsgemäßen Meßkartuschenaufnahme (20), in die eine Meßkartusche (5), mit zugehöriger Meßzelle (6), eingeschoben ist.

Durch die Öffnung (8) in der Meßkartusche (5) bzw. der Meßzelle (6) mit den Meß- und Bezugselektroden (11) kann man die Probenaufnahme (4) erkennen, die leicht mittels Spritzen bzw. Kapillaren (24) erreichbar ist.

Durch die bevorzugte Konstruktion der Meßzellen (6) als gespritzte Teile, z.B. aus glasklarem PVC oder ähnlichen Materialien, können ebenfalls die entsprechenden Strömkanäle (7, 9, 10, 12) leicht eingesehen und somit der korrekte Meßablauf vom Benutzer kontrolliert werden.

Im Längsschnitt durch die Meßkartuschenaufnahme (20) des schematisch angedeuteten Analysengerätes (1) der Fig. 3 wird gezeigt, wie die Meßkartusche (5) mit der Meßzelle (6) reversibel über die seitlichen, mechanischen Führungsschienen (26) der Meßkartusche (5) und zusätzlich über die inneren Kanten (27) des Ventilblocks (2) geführt, in die Aufnahme (20) einbringbar ist.

Die in der Fig. 3 gezeigte Anordnung der elektrischen Ableitungen, der Meßelektroden (18) in Form einer Leiterplatte und dem Steckkontakt (21), in der auch das in der Fig. 1 gezeigte elektronische Speichermedium (16) sowie das im Analysensystem (1) integrierte Gegenstück (21) zum Steckkontakt (19) der Ableitung (28) untergebracht ist, ermöglicht den Aufbau eines Farraday-Käfigs, der sich besonders positiv auf die Eliminierung elektrischer Störungen während der Messungen auswirkt

Die Fig. 4 ist eine Detailzeichnung entsprechend dem Längsschnitt in der Fig. 3, wobei die Fig. 4 zeigt, wie die Meßkartusche (5) mit der Meßzelle (6) vom Ventilblock (2), mit der zugehörigen Probenaufnahme (4) kontaktiert wird.

Durch die hohe Miniaturisierung der Strömkanäle (9, 10, 12) mit Durchmessein von ≤ 2-3 mm und den in der erfindungsgemäßen Vorrichtung, der Septen (13) als Abschluß dieser Strömkanäle (9, 10, 12), die über kleine Kanülen (3) (Außendurchmesser ca. ≤ 2-3 mm) in den Ventilblöcken (2, 12) angestochen werden, ist eine exakte Führung der Meßkartusche (5) beim Ein- und Ausführen in die Meßkartuschenaufnahme (20) über mechanische Führungsschienen (26, 27), wie sie die Fig. 3 zeigt, sehr wichtig. Nur so können die Kanülen die Strömkanäle (7, 9, 12) sicher durch die Septen (13) anstechen und ohne Luftblasen kontaktieren.

Die Möglichkeit einer Variation dieser erfindungsgemäßen Vorrichtung wird in der Fig. 5 beschrieben. Hierbei liegt die Aufnahme (30) der Meßzelle außerhalb des computergesteuerten Monitors (1) und ist lediglich über Kabel (29) und den Eingangsstecker (28) für die Kabelverbindung (29) mit der Meßzellenaufnahme (30) und dem sterilen Meßelektrodeneinsatz (38) verbunden. Die eigentliche Meßzelle teilt sich hierbei in 2 Teile auf, der Meßzelle (6) analog der in der Fig. 1 mit den Strömkanälen (7, 9, 12), die auch hier über Septen (13) abgeschlossen werden, und dem Teil eines weiteren Strömkanals (36) der als Bestandteil dieser kombinierten Meßzette (6, 37) in die jeweiligen Extra-Korporalen-(Kreislauf-) Systeme eingebunden werden kann. Diese Einbindung erfolgt über spezielle (Schlauch-) Anschlüsse (33). Solche (Schlauch-) Anschlüsse (33) sind auch im Meßzellen-Teil (6), für den Anschluß von Spül- bzw. Kalibrierlösungen, auf der Seite des Ventilblocks (2), sowie im Pumpen-Ventilblock (15) für den Anschluß eines Abfallbehältnisses, vorgesehen.

Eine weitere Änderung dieser Variation der erfindungsgemäßen Vorrichtung ist, daß die Meß- und Bezugselektroden (11) aus der Fig. 1 bis 4, nicht direkt in der Meßzelle enthalten sind, sondern als ein eigenständiger Meß- und Bezugselektrodeneinsatz (38) über zwei Sterilschleußen (34) zunächst in die Kalibierungskammer (35) und danach in den Strömkanal (36) des Teils der Meßzelle (37) zur direkten Messung im Extra-Korporalen-(Kreislauf-) System eingebracht werden.

Dieser Aufbau erlaubt, daß alle Baugruppen sterilisiert werden können und über sterile Kalibrierlösungen alle Vorgänge direkt im sterilen Bereich durchgeführt werden können.

## Patentansprüche

1. Vorrichtung mit entsprechenden Meßkartuschen (5) oder Meßzellen (6) worin die Meßkartuschen (20, 30), bzw. Meßzellen (6, 37), zur Bestimmung biochemischer Meßparameter, in computergesteuerten Analysensystemen (1) austauschbar sind, und in denen eine Durchflußeinheit mit verschiedenartigen Meß- und Bezugselektroden (11) und der zugehörige aktive Teil des Strömkanals (10) der Durchflußeinheit in ein System von weiteren Strömkanäle (7, 9, 12) in der Meßkartusche (5), bzw. in der Meßzelle (6) integriert sind, **dadurch gekennzeichnet, daß**
• die Strömkanäle (7, 9, 10, 12) der Durchflußeinheit über wiederholt anstechbare Septen (13) hermetisch dicht gegen Gas und Wasserdampf- Durchtritt abgeschlossen sind, wenn die Meßkartusche (5), bzw. die Meßzelle (6) aus dem computergesteuerten Analysensystems (1) heraus genommen wurden - und
• wenn die die MeßBkartusche (5), bzw. die Meßzelle (6) in das computergesteuerte Analysensystem (1) eingeschoben ist, so daß die Strömkanäle (7, 9, 10, 12) der Durchflußeinheit durch die anstechbaren Septen (13) über die in den Ventilblöcken (2, 14) integrierten Kontaktnadein (3) angestochen und somit miteinander verbunden werden, und,
• daß eine in einem Ventilblock (2) eine integrierte Probenaufnahme (4) durch eine Öffnung (8) in der Meßkartusche (5) und der Meßzelle (6) hindurchreicht und somit von Außen für Spritzen oder Kapillaren zur Probeneingabe erreichbar ist, sowie,
• daß die Steuerung der Ventile und Pumpe zur Meßvorbereitung, Kalibrierung und Messung im gesamten vorgenannten Meßsystem über Daten erfolgt, die in der Meßkartusche in einem elektronischen Speichermedium (16), der Meßkartusche (5), bzw, der Meßzelle (6, 37), gespeichert sind, das über elektrische Kontakte (18, 21) mit dem computergesteuerten Analysensystem (1) in Verbindung steht.

2. Vorrichtung nach Anspruch 1, **dadurch gekennzeichnet, daß**
• die Meßkartusche (5), bzw. die Meßzelle (6) über einen elektrischen Kontakt (18, 32), in den ein les- und beschreibbares elektrisches Speichermedium (16) integriert ist, mit dem computergesteuerten Analysensystem (1), über ein im computergesteuertem Analysensystem (1) befindliche Kontaktgegenstück (18, 21, 31) derart in Verbindung steht,
• daß für unterschiedliche Meßkartuschen (5), und/oder Meßzellen (6) mit verschiedener Anzahl und Art von Meß- und Bezugselektroden (11) mit unterschiedlicher Größe des aktiven Strömkanals (10) alle für die Steuerung der Pumpe (16) und der Ventile in den Ventilblöcken (2, 15) benötigten Daten zur Positionierung der Proben- und Spül-, bzw. Kalibrierflüssigkeitsvolumina vorhanden sind.

3. Vorrichtung nach einem der Ansprüche 1 bis 2, **dadurch gekennzeichnet, daß** in einem Analysensystem (1) mehrere Aufnahmevorrichtungen (20) für Meßkartuschen (5), und/oder Meßzellen (6) vorhanden sind.

4. Vorrichtung nach einem der Ansprüche 1 bis 3, **dadurch gekennzeichnet, daß**
• zumindest eine Aufnahme (30) der Meßzelle (6, 37) außerhalb des Analysensystems (1) ist und über elektrische Kabelverbindungen (29) mit dem Analysensystem (1) verbunden ist, und daß
• in der Meßzelle (5, 37) ein separater Meß- und Bezugselektrodenblock (38) über zwei Sterilschleusen (34) in eine Kalibrierkammer (35) und den Strömkanal (36), der ein Zwischenstück eines Extra-Korporal-(Kreislauf-) Systems ist, eingebracht werden kann.

5. Vorrichtung nach Anspruch 4, **dadurch gekennzeichnet, daß** alle Bauteile der beschriebenen Vorrichtung, die Aufnahme (30) der Meßzelle mit den Ventilblöcken (2, 15), die Meßzelle (6, 37), sowie der Meß- und Bezugselektrodenblock (38) sterilisierbar und direkt in sterilen Bereichen einsetzbar sind.

## Claims

1. Apparatus with interchangeable measurement cartridges (5) or measurement cells (6) wherein measurement cartridges (20, 30) or measurement cells (6, 37) for the determination of bio-chemical measurement parameters, in computer controlled analysis systems (1), are exchangeable and integrated into the one inside a through flow unit with different measurement and reference electrodes (11) and related active sector of the flow cannula (10) of the through flow unit in a system with additional flow cannula (7, 9, 12) in the measurement cartridges (5) or in the measurement cell (6), wherein
• the flow cannulas (7, 9, 10, 12) of the flow unit must be hermetically sealed against gases and steam through the piercing septum's (13) when the measurement cartridges (5) or the measurement cells (6) are removed from the computer controlled analysis system (1) and
• when the measurement cartridge (5) or the measurement cell (6) are inserted into the computer controlled analysis system (1), so that the flow cannulas (7, 9, 10, 12) of the flow unit are pierced through septum's (13) by contact pins (3) integrated in the valve blocks (2, 14) and are connected to each other, and,
• that one of the valve block (2) integrated sample reception (4) fits through an opening (8) into the measurement cartridge (5) and the measurement cell (6) and is therefore reachable from outside for hypothermic needles or capillaries for sample input, as well as,
• that the valve and pump control for measurement preparation, calibration and measurement is implemented to the complete named measurement system by data, that stored in an electric storage medium (16), the measurement cartridge (5) or/and the measurement cells (6, 37) which is connected to the computer controlled analysis system (1) and in contact by electric contacts (18, 21).

2. Apparatus according to claim 1, wherein
• the measurement cartridge (5) and the measurement cell (6) are combined by electrical contact (18, 32), that has a read/writeable electrical storage medium (16) integrated, to the computer controlled analysis system (1), and is in contact by means of a contact counterpart (18, 21, 31) with the computer controlled analysis system (1),
• for different measurement cartridges (5) or/and measurement cells (6) with a different number of types of measurement and reference electrodes (11) with different sizes of the active flow cannula (10) that the necessary data is available for positioning the sample and rinsing or calibration liquid volume required for controlling the pump (16) and the valve in the valve blocks (2, 15).

3. Apparatus according to one of the claims 1 to 2, wherein an analysis system (1) has several receptions (20) for measurement cartridges (5) and, /or for measurement cells (6).

4. Apparatus according to one of the claims 1 to 3 wherein
• a minimum of one reception (30) of the measurement cells (6, 37) is outside of the analysis system (1) and combined by electrical cable connections (29) with the analysis system (1), and that
• in the measurement cells (5, 37) a separate measurement and reference electrode block (38) can be fitted by two sterile gateways (34) to calibration chamber (35) and flow cannula (36), which is an intermediate unit of a extra-corporal- (closed circuit) system.

5. Apparatus according to claim 4, wherein all construction parts of the described apparatus, the reception (30) of the measurement cell with the valve unit (2, 15), the measurement cells (6, 37) as well as the measurement and reference electrode block (38) can be sterilized and used directly in the sterile sector.

## Revendications

1. Des dispositifs comportant des cartouches conformes de mesure (5) ou des cellules de mesure (6), où les cartouches de mesure (20, 30), voire les cellules de mesure (6, 37), destinées à la détermination de paramètres biochimiques de mesure, sont interchangeables dans des systèmes d'analyse commandés par ordinateur interposé (1), et dans lesquelles, dans une unité d'écoulement dotée d'électrodes de mesure et de référence de différentes catégories (11) et l'élément actif faisant partie du conduit d'écoulement (10) de l'unité d'écoulement, sont intégrées dans un système d'autres conduits d'évacuation (7, 9, 12) dans la cartouche de mesure (5), ou la cellule de mesure (6) se distinguent en ceci que **:**
- les conduits d'évacuation (7, 9, 10, 12) de l'unité d'écoulement par le biais de septum susceptibles d'être percés à plusieurs reprises (13) sont hermétiquement fermés, ne laissant passer ni la vapeur de gaz ni la vapeur d'eau une fois que la cartouche de mesure (5), voire la cellule de mesure (6) a été retirée du système d'analyse commandé par ordinateur interposé (1) - et,
• lorsque la cartouche de mesure (5), voire la cellule de mesure (6) est introduite dans le système d'analyse commandé par ordinateur interposé (1), de telle sorte que les conduits d'évacuation (7, 9, 10, 12) de l'unité d'écoulement soient percés au travers des septum susceptibles d'être percés (13), par le biais des aiguilles de contact (3) intégrées dans les blocs de régulation et de distribution (2, 14) , ce qui permet aux conduits d'être reliés entre eux et
• qu'un captage d'échantillons (4) intégré dans un bloc de régulation et de distribution (2), utilisant une ouverture (8) pratiquée dans la cartouche de mesure (5) et la cellule de mesure (6) traverse le dispositif tout entier et soit ainsi accessible depuis l'extérieur pour les seringues ou tubes capillaires, permettant ainsi d'introduire des échantillons ainsi que,
• la commande des soupapes et pompes en vue de la préparation du processus de mesurage, du calibrage et du mesurage dans l'ensemble du système de mesure précité, est pratiquée à l'aide de données qui sont mémorisées dans la cartouche de mesure, sur un support d'enregistrement (16), électronique de la cartouche de mesure (5), ou/et de la cellule de mesure (6, 37). Ce support d'enregistrement est relié, par le biais de contacts électriques (18, 21), au système d'analyse (1) commandé par ordinateur interposé.

2. Le dispositif selon le droit au brevet se distingue encore en ceci que :
• La cartouche de mesure (5), et/ou la cellule de mesure (6), à travers un contact électrique (18, 32), dans lequel un support d'enregistrement (16) lisible et imprimable est intégré, est reliée de telle façon au système d'analyse commandé par ordinateur interposé (1), par le biais d'un pendant (18, 21, 31) au dispositif de contact se trouvant dans le système d'analyse commandé par ordinateur interposé (1)
• que, pour différentes cartouches de mesure (5) et/ou cellules de mesure (6) avec différents nombre et catégories d'électrodes de mesure et de référence (11), présentant des tailles différentes de conduits actifs d'évacuation (10), toutes les données requises pour la commande de la pompe (16) et des soupapes dans les blocs de régulation et de distribution (2, 15), aux fins de positionnement des volumes de liquides des échantillons ainsi que des calibrages et rinçages, sont en place.

3. Le dispositif selon l'un des droits 1 à 2 au brevet se distingue en ceci que plusieurs dispositifs de captage (20) pour cartouches de mesure (5) et/ou cellules de mesure (6) se trouvent dans un système d'analyse (1).

4. Le dispositif selon l'un des droits 1 à 3 au brevet se distingue en ceci que
Au moins un captage (30) de la cellule de mesure (6, 37) se trouve à l'extérieur du système d'analyse (1) et est relié au système d'analyse (1) par des embranchements électriques par câbles (29) et que
• dans la cellule de mesure (5, 37), un bloc indépendant d'électrodes de référence et de mesure (38) peut être placé dans une chambre de calibrage (35), en passant par deux canaux stériles de transfert (34) , et le conduit d'évacuation (36), qui compose un élément intermédiaire d'un système (circulaire) extérieur.

5. Le dispositif selon le droit 4 au brevet se distingue en ceci que tous les éléments de construction du dispositif objet de la description, le captage (30) de la cellule de mesure avec les blocs complets de régulation et de distribution (2, 15), la cellule de mesure (6, 37), ainsi que le bloc d'électrodes de mesure et de référence (38) peuvent être stérilisés et utilisés directement dans des environnements stériles.
